# EUROPÄISCHE PATENTSCHRIFT

(11) **EP 1 405 592 B1**
(45) Veröffentlichungstag und Bekanntmachung des Hinweises auf die Patenterteilung: **07.01.2009**
(21) Anmeldenummer: 03020827.6
(22) Anmeldetag: 13.09.2003
(51) Int. Cl.: A61B 5/022

(54) **Verfahren zur Blutdruckmessung und Blutdruckmessgerät mit Benutzerführung**
Method for measuring blood pressure and blood-pressure measuring apparatus with user guidance
Méthode pour mésurer la pression sanguine et appareil de mesure de la pression sanguine avec guidage d'utilisateur

(30) Priorität: 02.10.2002 DE 10246255
(43) Veröffentlichungstag der Anmeldung: 07.04.2004
(73) Patentinhaber: KAZ Europe SA, 1003 Lausanne (CH)
(72) Erfinder: Freund, Dirk, 65779 Kelkheim (DE); Harttmann, Brigitte, 65527 Niedernhausen (DE); Hollinger, Stefan, 61476 Kronberg (DE); Rönneberg, Gerrit, 64289 Darmstadt (DE); Schnak, Fred, 61476 Kronberg (DE); Wunder, Dieter, 63679 Schotten (DE); Simeth, Martin, 61462 Königstein (DE); Zellermeyer, Siegfried, 60437 Frankfurt (DE); Müller, Waltraud, 65830 Kriftel (DE)
(74) Vertreter: Grünecker, Kinkeldey, Stockmair & Schwanhäusser Anwaltssozietät

(56) Entgegenhaltungen:
- WO-A-01/49171
- DE-A- 19 757 974
- US-A- 5 778 879

## Beschreibung

Die Erfindung betrifft ein Verfahren mit den Merkmalen des Oberbegriffs des Anspruchs 1. Die Erfindung betrifft ebenfalls ein Blutdruckmeßgerät mit den Merkmalen des Oberbegriffs des Anspruchs 10.

Eine der Hauptursachen für eine oftmals unzureichende Reproduzierbarkeit und mangelnde Meßgenauigkeit von Blutdruckmessungen am Handgelenk liegt in der hohen Empfindlichkeit der Messung auf Schwankungen der Meßposition relativ zur individuellen Lage des Herzens. Nur bei einer Meßposition auf Herzhöhe können exakte Blutdruckmeßergebnisse erhalten werden. Im Falle einer von der Herzhöhe abweichenden Meßposition verfälscht die hydrostatische Druckdifferenz das Ergebnis um etwa 0,75 mm Hg je 1 cm Höhenunterschied zwischen Meßposition und Herzhöhe. Bei einem Blutdruckmeßgerät zur Anlage am Unterarm bzw. Handgelenk oder Finger kann somit bei einer fehlerhaften Meßposition ein gravierender Meßfehler das Ergebnis verfälschen. Die mangelhafte Lage während des Meßvorgangs führt damit zu einem systematischen Meßfehler.

Zur Vermeidung dieses Problems ist aus der DE 197 57 974 A1 ein Verfahren und ein Blutdruckmeßgerät der eingangs genannten Art bekannt, bei dem der Neigungswinkel des Unterarms vor der Blutdruckmessung bestimmt wird. Das Ergebnis dieser Positionserfassungseinrichtung wird dem Benutzer auf der gleichen Anzeigeeinrichtung mitgeteilt, auf der später auch die Ergebnisse der Blutdruckmessung angezeigt werden. Zur Benutzerführung werden Zeichen für bevorstehende erforderliche Benutzeraktionen abhängig vom Ergebnis der Auswertung der aktuellen Position der Manschette dargestellt, bis eine Zielposition relativ zur Herzhöhe erreicht ist. Hierzu wird die bevorstehende erforderliche Benutzeraktion durch abwärts oder aufwärts gerichtete Pfeile auf der Anzeigeeinrichtung dargestellt. Mit der Bedienungsanleitung oder mit der Verkaufsverpackung des Blutdruckmeßgerätes wird der Benutzer darüber informiert, daß eine bestimmte Meßposition, z. B. mit angewinkeltem Unterarm vor dem Brustkörper während der Neigungsmessung und auch der Blutdruckmessung einzunehmen ist.

Ein weiteres Blutdruckmeßgerät mit Neigungssensor ist aus der DE 199 63 633 A1 sowie aus der WO 01/49171 A1 bekannt.

In der Praxis hat sich jedoch gezeigt, daß der durchschnittliche Benutzer trotz der Hinweise in der Bedienungsanleitung und auch auf der Verkaufsverpackung eine von der vorgesehenen Meßhaltung abweichende Meßposition einnimmt. Dies ist z. B. dadurch bedingt, daß vorausgegangene Blutdruckmeßgerätegenerationen und auch derzeitige Wettbewerbsprodukte zur oszillometrischen Blutdruckmessung mit einer Manschette am Handgelenk das Gehäuse an der Handflächenseite angeordnet hatten, wobei zur Messung der Ellenbogen auf einem Tisch abzustützen und der Unterarm so anzuwinkeln war, daß etwa die Herzhöhe erreicht wurde. Dabei ist es möglich, daß der Benutzer durchaus sein Blutdruckmeßgerät auf Herzhöhe hält, hierzu jedoch nicht den Unterarm entsprechend angewinkelt und in die vorgesehene Meßposition gebracht hat, sondern davon abweichend, z. B. den Oberarm mit dem Unterarm gemeinsam bewegt hat. Dies führt dazu, daß die Anzeige der Neigungswinkelmessung, deren Korrektheit davon abhängt, daß eine vorbestimmte Meßposition eingenommen wurde, beispielsweise einen nach oben gerichteten Pfeil anzeigt, so daß der Nutzer instruiert wird, möglicherweise über das bereits erreichte Herzniveau hinaus das Blutdruckmeßgerät anzuheben. Wenn die Positionserfassungseinrichtung als Neigungssensor ausgebildet ist, der die Neigung des Unterarms nur dann zutreffend wiedergibt, wenn der Unterarm von den vielen möglichen Freiheitsgraden zur Bewegung auf Herzhöhe in einer ganz bestimmten Weise, z. B. am Oberkörper entlang bewegt wird, wird dieses Problem vergrößert.

Zusammenfassend läßt sich feststellen, daß die bekannten Blutdruckmeßgeräte trotz Positionserfassungseinrichtung aufgrund von Fehlbedienung und einer fehlerhaft interpretierten vorgesehenen Benutzerführung das angestrebte Ziel einer besseren Reproduzierbarkeit und Erhöhung der Meßgenauigkeit nicht immer erreichen.

Zu einem vergleichbaren Ergebnis führt die Benutzerführung bei dem Blutdruckmeßgerät nach der US 5,778,879. Hierbei ist ebenfalls ein Neigungssensor am Blutdruckmeßgerät vorgesehen, wobei zur Benutzerführung abhängig vom Meßergebnis des Neigungssensors auf der Anzeigeeinrichtung entweder die Anweisung "Manschette ist zu hoch" oder "Manschette ist zu niedrig" ausgegeben wird. Auch hierbei werden die vielen Freiheitsgrade der Armgelenke nicht ausreichend berücksichtigt.

Es ist demgemäß eine Aufgabe der vorliegenden Erfindung, ein Verfahren und ein Blutdruckmeßgerät der eingangs genannten Art bereitzustellen, das den Benutzer besser zu einer vorgesehenen Meßposition führt, so daß die Meßergebnisse tatsächlich auf Herzhöhe erfolgen und damit genauer und reproduzierbar sind.

Diese Aufgabe wird durch ein Verfahren mit den Merkmalen des Anspruchs 1 und durch ein Blutdruckmeßgerät mit den Merkmalen des Anspruchs 10 gelöst. Erfindungsgemäß stellt die Benutzerführung die bevorstehende erforderliche Benutzeraktion unmittelbar in Bezug zu den bewegenden Körperteilen des Benutzers dar, um die Zielposition zu erreichen.

In vorteilhafter Weiterbildung des Verfahrens und des Blutdruckmeßgerätes ist/wird über die Anzeigeeinrichtung ein Oberkörper mit einem angewinkelten Unterarm dargestellt. Der Oberkörper kann dabei als schematisches Torsopiktogramm dargestellt sein. Ein Mikroprozessor des Blutdruckmeßgerätes kann hierzu entweder entsprechend programmiert sein, so daß eine pixelgesteuerte Anzeige auf der Flüssigkristallanzeigeeinrichtung erfolgt, die den Torso zeigt. Alternativ ist eine Permanentbedruckung in der Flüssigkristallanzeige oder an dieser möglich, so daß unabhängig davon, ob das Blutdruckmeßgerät bereits eingeschaltet oder mit einer Stromzufuhr versehen ist, für den Benutzer ein Hinweis auf die Meßposition gegeben wird.

In vorteilhafter Weiterbildung weist die Benutzerführung zur Erreichung der Zielposition auf der Anzeigeeinrichtung einen passiven und einen animierten, dynamischen Bestandteil auf. Dabei ist der passive Bestandteil ein fester unbeweglicher und der animierte Bestandteil beweglich. Auf diese Weise kann dem Benutzer eine völlig eindeutige Benutzerführung bereitgestellt werden, weil auf der Anzeigeeinrichtung verdeutlicht wird, welche Körperteile ruhig zu halten sind und welche Körperteile, namentlich der Unterarm mit der Manschette am Handgelenk oder am Finger, entsprechend zu bewegen ist ,damit als Zielposition das Herzniveau erreicht wird.

In vorteilhafter Weiterbildung ist die Anzeigeeinrichtung derart ausgebildet, daß nicht nur die erforderliche Benutzeraktion beispielsweise in Form von Pfeilen dargestellt wird, sondern auch die einzunehmende Meßposition und die aktuelle Meßposition darstellbar ist. Auf diese Weise wird der Benutzer in sehr eindeutiger Weise instruiert, so daß der Unterarm mit der Manschette zur Blutdruckmeßung von den vielen möglichen Freiheitsgraden, mit denen er bewegt werden kann, um das Herzniveau zu erreichen, auf die gewünschten Bewegungen und Haftungen reduziert instruiert wird.

Vorteilhafterweise ist als passiver Bestandteil die einzunehmende Meßposition mit einem Oberkörper und einem angewinkelten Unterarm angezeigt, wobei innerhalb des passiven Bestandteils ein animierter Unterarm abhängig vom Ergebnis der Auswertung der aktuell erfaßten Position der Manschette angezeigt wird, der die aktuelle Meßposition wiedergibt. Beispielsweise wird als passiver Bestandteil die Permanentbedruckung in der Flüssigkristallanzeige mit einem Oberkörper mit angewinkeltem Unterarm dargestellt, wobei der Unterarm derart animiert wird, daß er um das Ellenbogengelenk unterschiedliche Winkelpositionen annimmt, die unterhalb, oberhalb oder auf Herzniveau sind. Es versteht sich, daß der Unterarm dargestellt wird, an dem das Blutdruckmeßgerät mit Manschette getragen wird. Der bewegliche Unterarm kann auf diese Weise abhängig vom erfaßten Signal der Positionserfassungseinrichtung die aktuelle Meßposition wiedergeben. Die bevorstehende erforderliche Benutzeraktion, damit die Zielposition auf Herzhöhe erreicht wird, kann zusätzlich durch Pfeile am animierten Unterarm oder durch ein blinkendes Herz bei Erreichen der Zielposition dargestellt werden.

In vorteilhafter Weiterbildung wird mit der Blutdruckmessung erst dann automatisch begonnen, ohne daß ein weiterer Bedienungsknopf zu drücken ist, wenn entweder die Zielposition für die korrekte Meßhaltung erreicht wurde, oder eine bestimmte Zeit verstrichen ist.

Vorteilhafterweise gibt die Anzeigeeinrichtung abhängig vom Ergebnis der Auswertung der aktuell erfaßten Position der Manschette eine Textanweisung oder Sprachanweisung und damit eine unmittelbare Anweisung für die zu positionierenden Körperteile aus. Beispielsweise kann der Benutzer eindeutig per Textanweisung instruiert werden: "Meßarm an die Brust legen", "Meßarm absenken", "Arm halten" oder "Arm weiter anwinkeln". Auf diese Weise ist Bezug auf die zu bewegenden Körperteile genommen, so daß für den Benutzer ebenfalls in eindeutiger Weise eine Instruktion für die korrekte Meßhaltung bereitgestellt ist.

In weiterer vorteilhafter weise ist die Benutzerführung derart ausgebildet, daß der Torso mit dem Meßarm im Regelfall spiegelbildlich zum Benutzer dargestellt ist. In den meisten Fällen hat sich gezeigt, daß das Blutdruckmeßgerät - obwohl für links und rechts zur Anlage am Unterarm geeignet - am linken Unterarm getragen wird. Darauf abgestimmt, wird in der Anzeigeeinrichtung spiegelbildlich der Meßarm am Torso dargestellt. Dies vermittelt dem Benutzer den Eindruck, daß wie vor einem Spiegel stehend, die dargestellte einzunehmende Meßhaltung lediglich nachzuahmen ist.

Weitere vorteilhafte Merkmale der Erfindung sind in den Unteransprüchen dargestellt.

Weitere Ziele, Vorteile, Merkmale und Anwendungsmöglichkeiten der vorliegenden Erfindungen ergeben sich aus der nachfolgenden Beschreibung von 4 Ausführungsbeispielen anhand der Zeichnungen. Dabei bilden alle beschriebenen und/oder bildlich dargestellten Merkmale für sich oder in beliebiger sinnvoller Kombination den Gegenstand der vorliegenden Erfindung, auch unabhängig von ihrer Zusammenfassung in den Patentansprüchen oder deren Rückbeziehung.

Es zeigen:
- Fig. 1: eine schematische räumliche Darstellung des erfindungsgemäßen Blutdruckmeßgerätes befestigt an der oberen Schmalseite des geschnitten dargestellten Handgelenks eines Benutzers,
- Fig. 2: eine schematische Darstellung eines Benutzers mit angelegtem Blutdruckmeßgerät nach Fig. 1 in korrekter Meßposition,
- Fig. 3: eine Permanentbedruckung einer Anzeigeeinrichtung des Blutdruckmeßgeräts nach Fig. 1 zur Benutzerführung nach einer ersten Ausführungsform,
- Fig. 4: die Anzeigeeinrichtung mit Benutzerführung nach Fig. 3 bei zu hoher Meßposition,
- Fig. 5: die Anzeigeeinrichtung mit der Benutzerführung nach Fig. 3 bei etwas zu hoher Meßposition,
- Fig. 6: die Anzeigeeinrichtung mit der Benutzerführung nach Fig. 3 kurz vor Erreichen der Herzposition,
- Fig. 7: die Anzeigeeinrichtung mit der Benutzerführung nach Fig. 3 bei zu niedrigem Unterarm,
- Fig. 8: die Anzeigeeinrichtung mit der Benutzerführung nach Fig. 3 bei etwas zu niedrigem Unterarm,
- Fig. 9: die Anzeigeeinrichtung mit der Benutzerführung nach Fig. 3 kurz vor Erreichen des Herzniveaus,
- Fig. 10: die Anzeigeeinrichtung mit einer Benutzerführung nach Fig. 3 bei erreichter und dauerhaft gehaltener Herzposition des Unterarms,
- Fig. 11a: die Benutzerführung auf der Anzeigeeinrichtung bei zu hoher Meßposition des Unterarms nach einer zweiten Ausführungsform,
- Fig. 11b: die Benutzerführung nach Fig. 11a bei erreichter Herzhöhe,
- Fig. 11c: die Benutzerführung nach Fig. 11a bei zu niedriger Meßposition,
- Fig. 12a: die Benutzerführung der Anzeigeeinrichtung bei zu hoher Meßposition nach einer dritten Ausführungsform,
- Fig. 12b: die Benutzerführung nach Fig. 12a bei Erreichen der Herzhöhe,
- Fig. 12c: die Benutzerführung nach Fig. 12a bei zu niedriger Meßposition des Unterarms,
- Fig. 13a: die Benutzerführung der Anzeigeeinrichtung bei zu hoher Meßposition nach einer vierten Ausführungsform,
- Fig. 13b: die Benutzerführung nach Fig. 13a bei Erreichen der Herzhöhe,
- Fig. 13c: die Benutzerführung nach Fig. 13a bei zu niedriger Meßposition des Unterarms,
- Fig. 14: ein schematisches Flußdiagramm bei der Positionserfassung in einer ersten Variante des Blutdruckmeßgeräts nach Fig. 1 und
- Fig. 15: ein schematisches Flußdiagramm der Positionserfassung nach einer zweiten Variante des Blutdruckmeßgeräts nach Fig. 1.

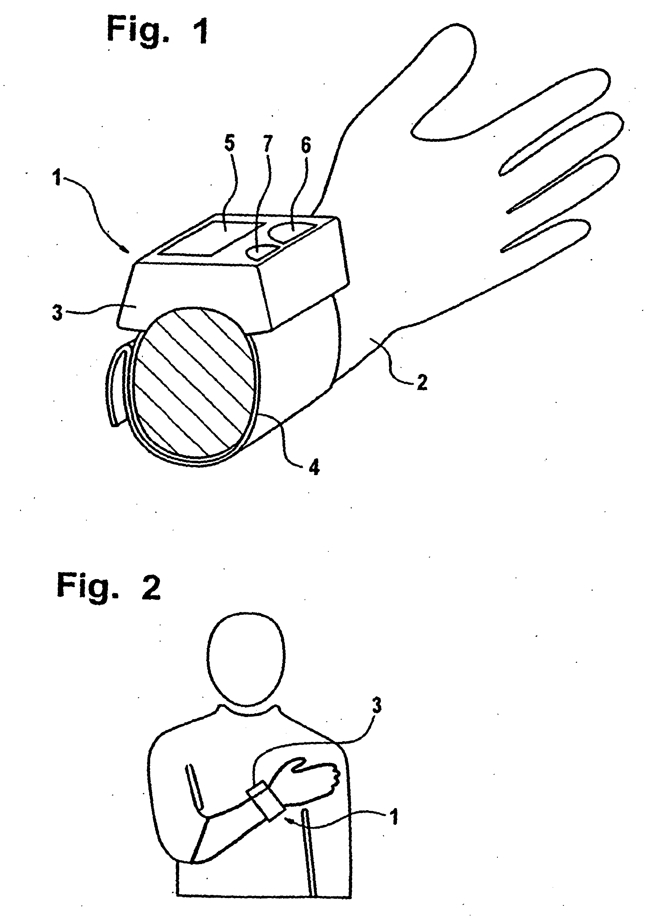

Das Blutdruckmeßgerät 1 nach Fig. 1 umfaßt eine Manschette 4, die an das Handgelenk oder in einer Variante (nicht dargestellt) an einen Finger des Benutzers angelegt werden kann. Das Gehäuse 3 des Blutdruckmeßgerätes ist bevorzugt an der Manschette 4 befestigt, wobei die Manschette und das Gehäuse derart ausgebildet sind, daß das Gehäuse 3 des Blutdruckmeßgerätes 1 mit der Anzeigeeinrichtung 5 an der oberen Schmalseite des Handgelenks, also in Verlängerung des Daumens angeordnet ist. Damit ist u.a. gewährleistet, daß die Anzeigeeinrichtung 5 bei einer Meßposition nach Fig. 2 mit dem Unterarm vor dem Oberkörper anliegend ablesbar ist.

Die Anzeigeeinrichtung 5 ist an der oberen Flachseite des Gehäuses 3 angeordnet, an einer Fläche des Gehäuses, an der der Ein/Aus-Schalter 6 und die optionale Memorytaste 7 angeordnet sind. Die am Gehäuse 3 befestigte Manschette 4 weist vorzugsweise einen Kunststoffclip auf, der teilweise der Kontur eines Handgelenks gemäß ausgebildet ist. In der Manschette 4 ist eine Druckblase angeordnet, die von einer Pumpe im Gehäuse 3 derart aufblasbar ist , daß eine Arterie an der Innenseite des Handgelenks abdrückbar ist. Das Blutdruckmeßgerät weist im übrigen alle standardmäßigen Komponenten auf, die zur Blutdruckmeßung nach dem oszillometrischen Prinzip erforderlich und bei Handgelenksblutdruckmeßgeräten mittlerweile üblich sind.

Das Blutdruckmeßgerät weist in allen hier dargestellten Ausführungsformen ferner eine Positionserfassungseinrichtung auf, die z.B. als Neigungssensor ausgebildet ist. Der Neigungssensor kann beispielsweise ein Pendel sein, dessen Position elektronisch detektiert wird.

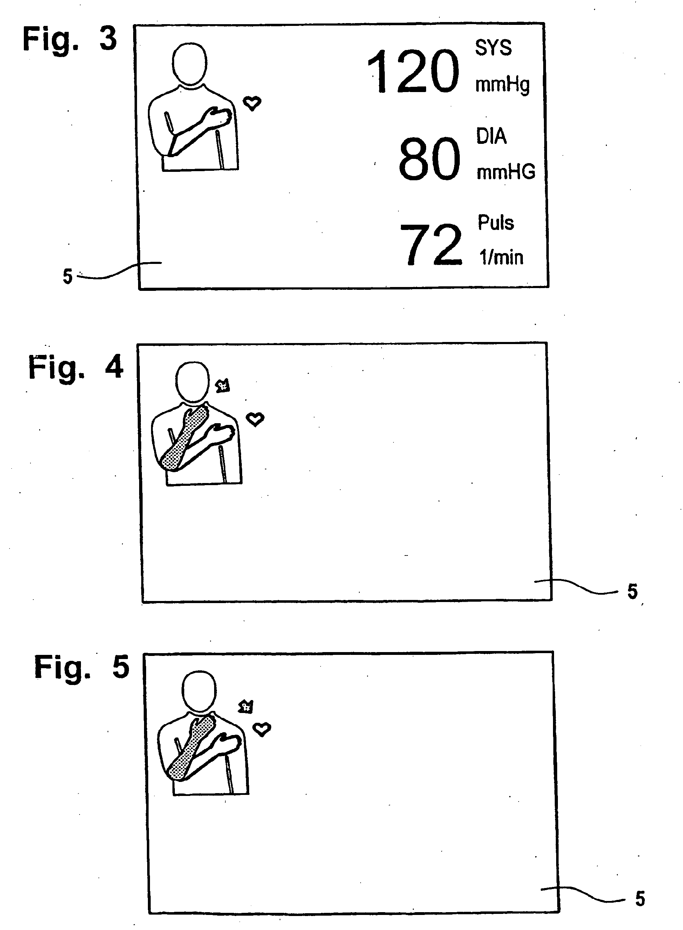

An der Anzeigeeinrichtung 5 wird sowohl das Ergebnis der Blutdruckmeßung als auch die Benutzerführung angezeigt. Als Ergebnis der Blutdruckmeßung wird vorzugsweise der systolische und der diastolische Blutdruck in mm Hg als auch der Puls angezeigt. Die Anzeigeeinrichtung 5 ist als Flüssigkristallanzeige, wie sie auch bei Handys oder Laptops zum Einsatz kommt, ausbildbar. Unmittelbar neben den Blutdruckmeßergebnissen ist schematisch ein Torsopiktogramm wie in Fig. 3 gezeigt, permanent in der Anzeigeeinrichtung aufgedruckt. Damit sind Instruktionen für den Benutzer unabhängig davon, ob Batterien eingelegt sind, oder der Einschalter bereits betätigt wurde, zur optimalen Körperhaltung jederzeit gegeben. Das Torsopiktogramm zeigt bevorzugt einen Oberkörper mit angewinkeltem Arm, wobei der Meßarm, an dem das Blutdruckmeßgerät angelegt ist ,farblich oder anderweitig hervorgehoben ist. Die Meßposition wird zusätzlich durch ein Herzsymbol in gleicher Darstellung wie der Meßarm und benachbart zu diesem illustriert.

Vorzugsweise erfolgt die Bedruckung an der unteren der beiden Glasscheiben der Flüssigkristallanzeige, wenn die Anzeigeeinrichtung 5 als solche ausgebildet ist.

Nach dieser ersten Ausführungsform ist der dargestellte Oberkörper als Torsopiktogramm zur Darstellung der einzunehmenden Meßhaltung passiv, permanent bzw. unveränderlich an der Anzeigeeinrichtung 5 dargestellt. Die für die Meßhaltung relevanten Körperteile werden zumindest gezeigt. Die einzunehmende Meßhaltung kann alternativ zur permanenten Bedruckung auch durch eine dynamische Einblendung der Zielposition in der Anzeigeeinrichtung angezeigt werden, solange, bis die Zielposition erreicht ist. Eine Kontrolleinheit, wie z.B. ein Mikroprozessor steuern die Anzeigeeinrichtung entsprechend an. Die Zielposition kann dabei durch Farben, Symbole oder Linien hervorgehoben werden. Alternativ dazu ist eine verbale Beschreibung der Meßhaltung möglich, die als Klartextanzeige auf einer textfähigen Anzeigeinrichtung oder per Sprachausgabe kommuniziert wird, z.B.: "Legen Sie Ihre linke Hand an Ihre rechte Schulter" oder "Arm an Brust anlegen".

Die aktuelle Position de Benutzers wird vom Blutdruckmeßgerät mittels der Postionserfassungseinrichtung ermittelt. Die Darstellung der aktuellen Position erfolgt vorzugsweise grafisch ebenfalls in Form eines Piktogramms, wobei mindestens die für die Meßhaltung relevanten Körperteile in ihrer aktuellen Position dargestellt werden. Dies kann durch eine dynamische Einblendung des relevanten Körperteils in seiner Position über einem statisch (bzw. passiven oder permanenten) aufgebrachten Piktogramm oder durch die Darstellung des kompletten Piktogramms in einem grafikfähigen Display erfolgen. Alternativ ist eine verbale Beschreibung der aktuellen Position möglich, die als Klartextanzeige auf einem textfähigen Display oder per Sprachausgabe kommuniziert: "Ihr Handgelenk befindet sich unterhalb der Herzhöhe".

Zusätzlich zur Darstellung der einzunehmenden Meßhaltung und zur Darstellung der aktuellen Position des Benutzers wird die erforderliche Benutzeraktion durch Zeichen wie z.B Pfeile dargestellt, damit der Unterarm, an dem das Blutdruckmeßgerät befestigt ist, in die korrekte Position bewegt wird. Nachdem das Blutdruckmeßgerät die aktuelle Position des Unterarms des Benutzers mittels der Positionserfassungseinrichtung ermittelt hat, kann es die zum Erreichen der Zielposition mit der Manschette auf Herzhöhe nötige Aktion bestimmen. Diese Aktion wird vorzugsweise grafisch in Form eines Piktogramms dargestellt, wobei ein oder mehrere Pfeile die Richtung der auszuführenden Bewegung von der aktuellen in die Zielposition beschreiben oder die auszuführende Bewegung in Form eines animierten Piktogramms auf einem grafikfähigen Dislay bzw. Anzeigeeinrichgung gezeigt wird oder die Bewegung durch animierte Pfeilsymbole dargestellt wird. Alternativ ist eine verbale Beschreibung der auszuführenden Bewegung möglich, die als Klartextanzeige auf einem textfähigen Display oder per Sprachausgabe kommuniziert: "Arm anwinkeln" oder "Arm stärker anwinkeln".

Bei der ersten Ausführungsform nach den Fig. 3 bis 10 ist zusätzlich zur statischen bzw. passiven Anzeige der einzunehmenden Meßhaltung die aktuelle Position des Benutzers durch einen zweiten Unterarm dargestellt, der farblich oder durch andere kontrastierende Mittel vom Unterarm unterscheidbar ist, der die ideale Meßposition auf Herzhöhe anzeigt. Für den Fall, daß die Positionserfassungseinrichtung detektiert, daß die Meßposition zu hoch ist, weil der Unterarm zu stark angewinkelt ist, wird auf der Anzeigeeinrichtung gemäß Fig. 4 ein weiterer Unterarm dargestellt, der zu stark angewinkelt ist und dessen Meßposition zu hoch ist. Die erforderliche Benutzeraktion wird hier durch einen Pfeil in der gleichen Farbe oder im gleichen Kontrastierungsmittel dargestellt wie der zu hohe Meßarm, so daß für den Benutzer mühlelos erkennbar ist, daß der Meßarm zu hoch ist und etwas weniger stark anzuwinkeln ist. Der Pfeil weist hierzu in die Richtung ,in die der Unterarm zu bewegen ist. Der Pfeil wandert entsprechend der erfaßten Position des Unterarm entlang einer Kreisbahn, dessen Mittelpunkt etwa dem Ellenbogengelenk entspricht.

Fig. 4 zeigt den Unterarm des Benutzers bei starker Abweichung mit zu hoher Meßposition. In Fig. 5 ist eine Meßposition dargestellt, bei der der Benutzer seinen Unterarm bereits etwas auf die ideale Zielposition hin zubewegt hat, jedoch weiterhin den Unterarm absenken sollte. Gemäß dieser Ausführungsform ist die zu hohe Meßposition dadurch dargestellt, daß der Meßarm oberhalb der einzunehmenden Meßhaltung dargestellt ist, wobei die erforderliche Benutzeraktion durch einen Pfeil dargestellt ist, der auf das Herzsymbol zu gewandert ist, so daß der Benutzer durch den verringerten Abstand zum Herzsymbol eine Rückmeldung erhält, daß seine Bewegungsrichtung zur einzunehmenden Meßhaltung führt.

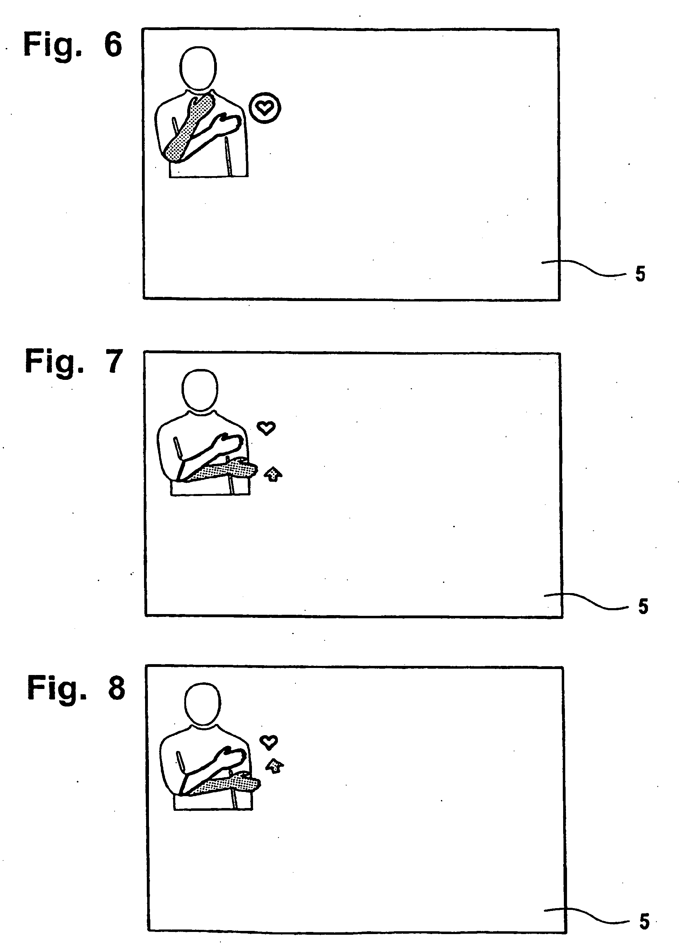

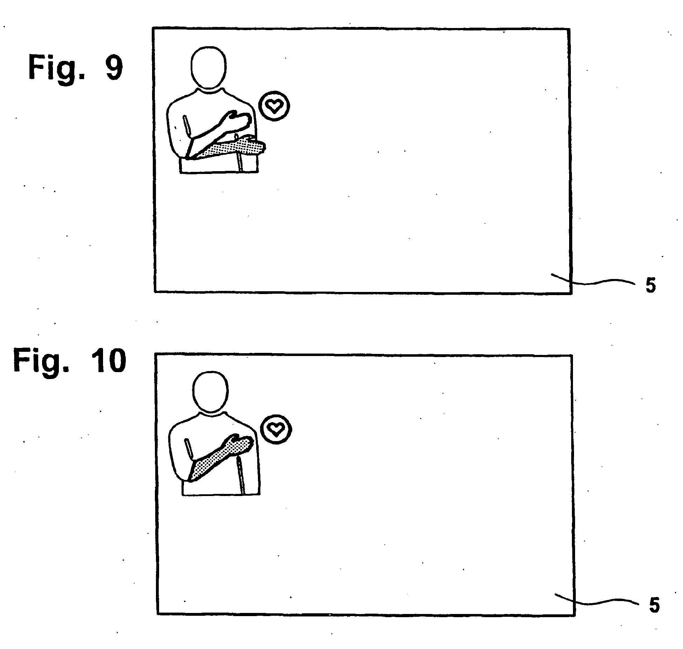

Sobald der Benutzer anschließend die einzunehmende Meßhaltung fast erreicht hat, wird dies dadurch dargestellt - siehe Figuren 6 und 9 -, daß der Unterarm für die Darstellung der aktuellen Position des Benutzers immer noch eine Meßposition als zu hoch anzeigt, jedoch als erforderliche Benutzeraktion kein Pfeilsymbol, sondern eine Umkreisung des Herzsymbols erfolgt. Natürlich kann dies auch anders dargestellt werden, indem z.B. das Herz vergrößert wird, seine Farbe ändert, blinkt oder anderweitig grafisch hervorgehoben wird, - insbesondere wenn dann die Herzposition erreicht ist. Erst wenn der Benutzer für etwas längere Zeit die einzunehmende Meßhaltung hält, wird zusätzlich zum eingekreisten Herzsymbol auch der Unterarm, der die einzunehmende Meßhaltung angibt, grafisch etwas anders hervorgehoben, sodaß für den Benutzer eindeutig ist, daß nun die optimale einzunehmende Meßhaltung erreicht ist (siehe Fig. 10). Alternativ kann die Erreichung der optimalen Meßhaltung unmittelbar angezeigt werden. Nach Erreichen der optima len Meßhaltung kann zusätzlich zur grafischen Anzeige ein akustisches Signal erfolgen. Die Blutdruckmessung startet nach Erreichen der optimalen Meßhaltung oder nach verstreichen einer bestimmten Zeitdauer von z.B. 10 bis 20 Sekunden nach drücken der Ein/Aus Taste automatisch.

Die Fig. 7, 8 und 9 zeigen die Benutzerführung analog zu den Fig. 4 bis 6, jedoch für den Fall, daß der Arm, an dem das Blutdruckmeßgerät befestigt ist, unterhalb der einzunehmenden Meßhaltung positioniert ist. Auch in diesem Fall wird bei dauerhafter Erreichung der einzunehmenden Meßhaltung das Herzsymbol umkreist hervorgehoben dargestellt, wobei sich der farblich hervorgehobene Unterarm für die einzunehmende Meßhaltung und der zweite dargestellte Unterarm für die aktuelle Position des Benutzers überdecken. In Weiterbildung dieser Ausführungsform kann der Unterarm in noch kleineren Bewegungsschritten gemäß der detektierten Position animiert werden.

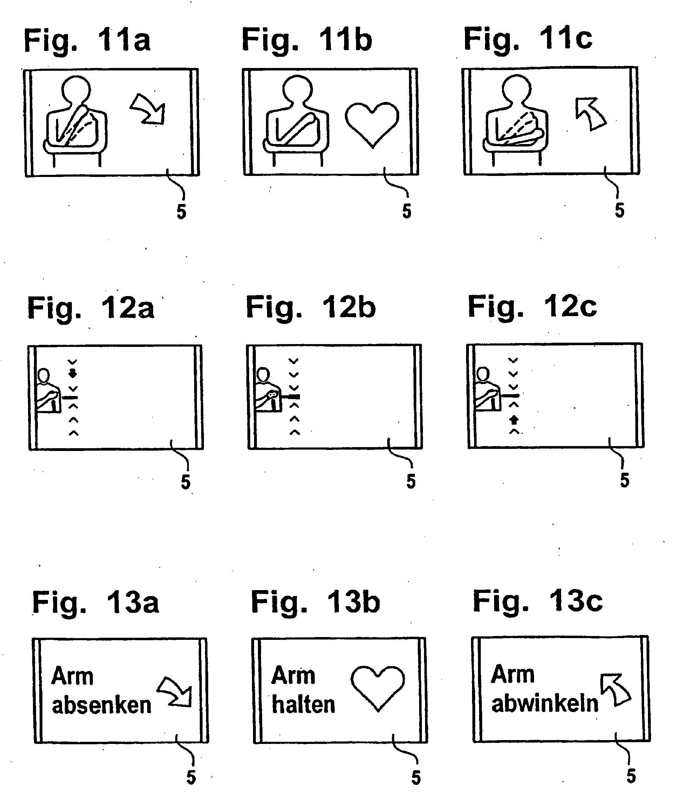

Die Figuren 11a bis c zeigen eine zweite Ausführungsform, bei der die komplette Benutzerführung ohne statische Anteile dynamisch, also animiert dargestellt ist.

In den Fig. 11a und 11c ist die Zielposition für die einzunehmende Meßhaltung auf Herzhöhe durch eine gestrichelte Armkontur dargestellt, während in Fig. 11a mit durchgezogener Linie die Armkontur für die aktuelle Position des Benutzers gemäß der Auswertung der Positionserfassungseinrichtung oberhalb der gestrichelten Armkontur dargestellt ist. Umgekehrt ist in Fig. 11c bei zu niedriger Meßposition mit durchgezogener Linie die Armkontur unterhalb der gestrichelten Armkontur für die einzunehmende Meßhaltung dargestellt. Die erforderliche Benutzeraktion wird durch Pfeilsymbole angedeutet, die in die Richtung deuten, in die der Arm zu bewegen ist. Bei Erreichen der einzunehmenden Meßhaltung überdecken sich die gestrichelte Armkontur für die einzunehmende Meßhaltung mit der Darstellung der aktuellen Position des Benutzers, wobei zusätzlich statt eines Pfeilsymboles ein Herzsymbol angezeigt wird.

Gemäß einer dritten Ausführungsform nach den Fig .12a bis c ist die einzunehmende Meßhaltung als Torsopiktogramm statisch bzw. permanent dargestellt, in dem das Display einfarbig bedruckt ist. Die erforderliche Benutzeraktion wird dadurch dargestellt, daß Pfeilsymbole auf unterschiedlicher Distanz relativ zum Herzniveau die notwendige Bewegungsrichtung des Unterarm mit dem Blutdruckmeßgerät anzeigen.

Bei der vierten Ausführungsform nach den Fig. 13a bis c wird die bevorstehende erforderliche Benutzeraktion bezogen auf die notwendige Bewegung des Meßarmes verbal durch eine dynamisch eingeblendete Klartextanzeige an der Anzeigeeinrichtung oder durch eine Sprachausgabe dargestellt. Zusätzlich zur Klartextanzeige wird entsprechend der erforderlichen Bewegungsrichtung ein Pfeilsymbol ausgegeben. Sobald die Herzhöhe erreicht ist, erhält der Benutzer gemäß Fig. 13b die Anweisung den Arm dauerhaft in dieser Position zu halten, wobei ein Herzsymbol angezeigt wird.

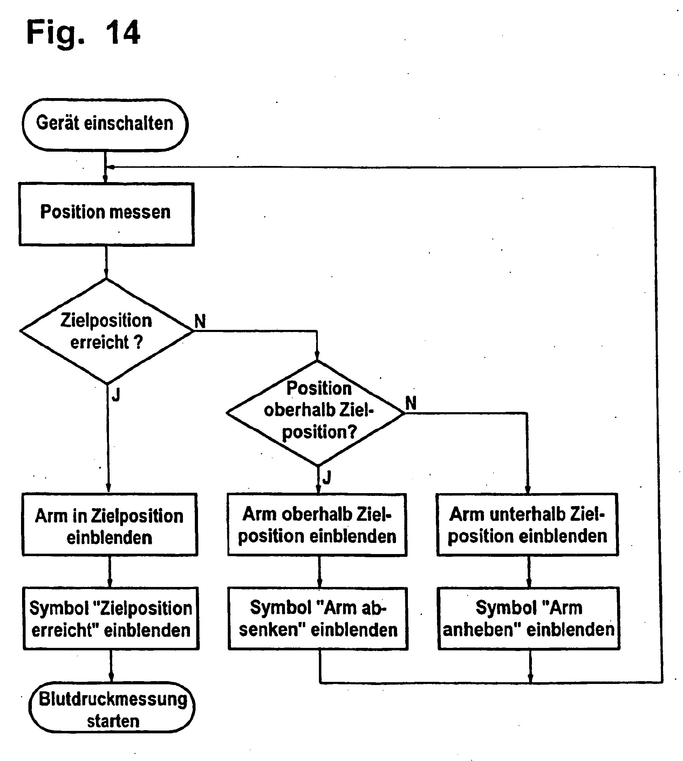

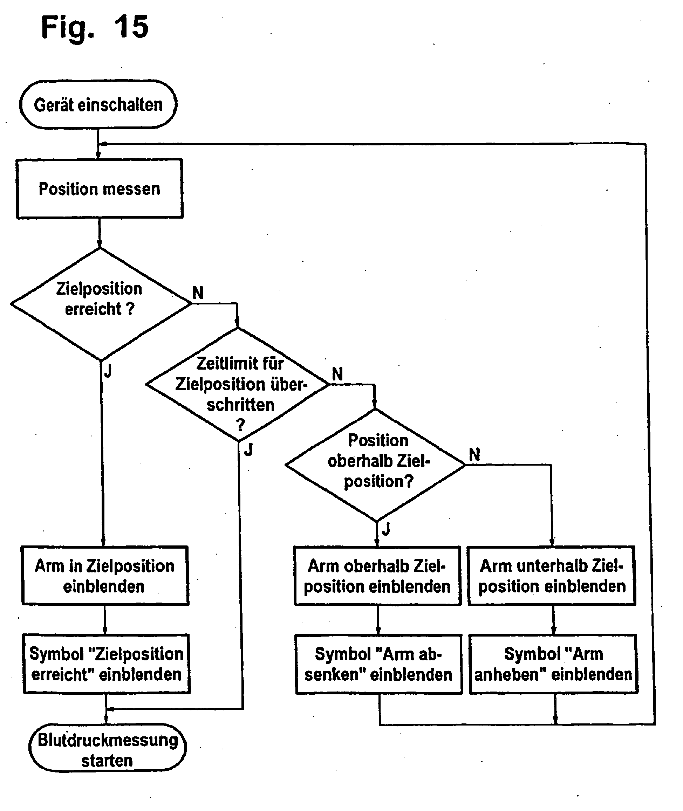

Im folgenden wird der Meßablauf kurz in zwei Varianten dargestellt. Grundsätzlich erfolgt die Positionserfassung zumindest vor der Blutdruckmessung. Der Benutzer drückt nur ein einziges Mal den Ein/Aus-Schalter, mit dem sowohl die Stromzufuhr für das Blutdruckmeßgerät eingeschaltet wird als auch die Positionsmessung und die Blutdruckmessung initiert wird. Bei beiden Varianten nach den Fig. 14 und 15 wird zunächst die Position des Unterarms, an dem das Blutdruckmeßgerät befestigt ist, mittels der Positionserfassungseinrichtung wie z.B. dem Neigungssensor erfaßt. Das Ergebnis wird in einem Controller bzw Mikroprozessor ausgewertet und mit einer standardisierten idealen Zielposition verglichen, die der einzunehmenden Meßhaltung entspricht. Ist die Zielposition nicht erreicht ("N" steht für Nein und "J" für Ja in Fig. 14 und 15), dann wertet der Controller anschließend aus, ob die aktuelle detektierte Position des Unterarms oberhalb oder unterhalb der Zielposition liegt. Dementsprechend erfolgt eine Ausgabe an der Anzeigeeinrichtung zur aktuellen Position des Benutzers wie oben beschrieben. Beispielhaft ist in den Fig. 14 und 15 ein Flußdiagramm dargestellt für die Ausführungsform mit Klartextausgabe. Alternativ ist der Meßablauf analog für die übrigen Ausführungsformen. Gemäß Fig. 14 wird die Routine zur Positionsmessung und des Vergleiches des Meßergebnisses mit der Zielposition solange durchgeführt, bis schließlich die Zielposition errreicht wird. Erst dann wird eine entsprechende Anzeige an der Anzeigeeinrichtung ausgegeben.

Gemäß der zweiten Variante in Fig. 15 wird ebenfalls die abweichende aktuelle Position von der Zielposition angezeigt bis die Zielposition erreicht ist, jedoch mit der Einschränkung, daß zur Positionsfindung ein maximales Zeitlimit von z.B. 15, 20 oder 30 Sekunden vorgesehen ist, so daß es für den Benutzer möglich ist, auch für den Fall, daß die Positionserfassungseinrichtung eine Position des Unterarms mit Blutdruckmeßgerät mißt, die gemäß der Auswertung von der Zielposition abweicht, der Zyklus zur Positionmessung beendet wird. In diesem Fall wird auf die Anzeige: - Zielposition erreicht - oder vergleichbarem verzichtet, wobei nach der Messung ein Hinweis auf die nicht korrekte Zielposition in der Anzeigeeinrichtung eingeblendet werden kann. Bei beiden Varianten gemäß Fig. 14 und 15 folgt unmittelbar nach dieser Positionserfassung, Anzeige und Auswertung die Blutdruckmessung, ohne daß der Benutzer einen weiteren Bedienknopf hierfür am Blutdruck betätigen müßte. Dies ist deshalb von Bedeutung, weil durch eine zusätzliche notwendige Betätigung eines Tasters zur Initiierung der Blutdruckmeßung eine Bewegung des anderen Armes erforderlich ist, die dazu führen könnte, daß die einzunehmende erreichte Meßhaltung wieder verlassen wird. Aus diesem Grund ist die gesamte Messung der Position mit dem Blutdruck und einschließlich des Einschaltens des Blutdruckmeßgerätes mit einem einzigen Ein/Aus-Schalter betätigbar.

## Patentansprüche

1. Verfahren zur Blutdruckmessung bei dem eine Manschette (4) an ein Handgelenk oder einen Finger eines Benutzers angelegt wird, mit Hilfe einer Positionserfassungseinrichtung eine Position der Manschette (4) erfaßt wird, die erfaßte Position der Manschette (4) ausgewertet wird, die Darstellung von grafischen Zeichen oder Ausgabe von verbalen Signalen für eine Benutzerführung von einer Kontrolleinheit kontrolliert wird und sowohl die Ergebnisse der Blutdruckmessung als auch die grafischen Zeichen oder verbalen Signale für die Benutzerführung auf einer Anzeigeeinrichtung (5) ausgegeben werden, wobei die grafischen Zeichen oder verbalen Signale über bevorstehende erforderliche Benutzeraktionen abhängig vom Ergebnis der Auswertung der aktuell erfaßten Position der Manschette (4) ausgegeben werden, damit eine Zielposition erreicht wird, **dadurch gekennzeichnet, daß** die Kontrolleinheit die Anzeigeeinrichtung (5) mit zur Zielposition abweichungsstärkeabhängigen grafischen Zeichen oder verbalen Signalen über die Bewegungsrichtung in Bezug auf die einzunehmende Zielhaltung ansteuert, wobei unterschiedliche grafische Zeichen oder verbale Signale bei unterschiedlich starker Abweichung von der Zielposition ausgegeben werden.

2. Verfahren nach Anspruch 1, **dadurch gekennzeichnet, daß** über die Anzeigeeinrichtung (5) ein Oberkörper mit einem angewinkelten Unterarm dargestellt wird.

3. Verfahren nach einem der Ansprüche 1 oder 2, **dadurch gekennzeichnet, daß** die Benutzerführung zur Erreichung der Zielposition auf der Anzeigeeinrichtung (5) einen passiven und einen animierten Bestandteil aufweist.

4. Verfahren nach zumindest einem der Ansprüche 1 bis 3, **dadurch gekennzeichnet, daß** die Anzeigeeinrichtung (5) neben der erforderlichen Benutzeraktion, die insbesondere animiert dargestellt ist, die einzunehmende Meßposition und die aktuelle Meßposition darstellt.

5. Verfahren nach zumindest einem der Ansprüche 3 oder 4, **dadurch gekennzeichnet, daß** als passiver Bestandteil die einzunehmende Meßposition mit einem Oberkörper und einem angewinkelten Unterarm angezeigt wird und daß innerhalb des passiven Bestandteils ein animierter Unterarm abhängig vom Ergebnis der Auswertung der aktuell erfaßten Position der Manschette (4) angezeigt wird, der die aktuelle Meßposition wiedergibt.

6. Verfahren nach zumindest einem der Ansprüche 1 bis 5, **dadurch gekennzeichnet, daß** die Blutdruckmessung erst dann automatisch gestartet wird, wenn die Zielposition für die korrekte Meßhaltung erreicht oder eine bestimmte Zeit verstrichen ist.

7. Verwahren nach zumindest einem der Ansprüche 1 bis 6, **dadurch gekennzeichnet, daß** der Start der Blutdruckmessung oder/und die Zielposition für eine korrekte Meßhaltung durch ein akustisches Signal angezeigt wird.

8. Verfahren nach Anspruch 7, **dadurch gekennzeichnet, daß** nach den Umständen des Zustandekommens des Starts der Blutdruckmessung ein je unterschiedliches akustischen Signal abgegeben wird.

9. Verfahren nach Anspruch 1, **dadurch gekennzeichnet, daß** die Anzeigeeinrichtung (5) abhängig vom Ergebnis der Auswertung der aktuell erfaßten Position der Manschette (4) eine Textanweisung oder Sprachanweisung und dabei eine unmittelbare Anweisung für die zu positionierenden Körperteile ausgibt.

10. Blutdruckmeßgerät mit einer Manschette (4) für eine Blutdruckmessung am Handgelenk oder Finger eines Benutzers, einer Positionserfassungseinrichtung zur Erfassung der Position der Manschette (4), einer Auswerteeinheit zur Auswertung der erfaßten Position der Manschette (4), einer Kontrolleinheit zur Kontrolle der Darstellung von grafischen Zeichen oder verbalen Signalen für eine Benutzerführung und einer die Ergebnisse der Blutdruckmessung und die grafischen Zeichen oder verbalen Signale für die Benutzerführung ausgebenden Anzeigeeinheit (5), wobei die Anzeigeeinrichtung (5) grafische Zeichen oder verbale Signale für die Benutzerführung über bevorstehende erforderliche Benutzeraktionen abhängig vom Ergebnis der Auswertung der aktuell erfaßten Position der Manschette (4) ausgebend vorgesehen ist, damit eine Zielposition erreichbar ist, **dadurch gekennzeichnet, daß** die Anzeigeeinrichtung (5) mit unterschiedlichen, zur Zielposition abweichungsstärkeabhängigen, grafischen Zeichen oder verbalen Signalen ansteuerbar und diese ausgebend vorgesehen ist, und die Kontrolleinheit die Anzeigeeinheit (5), mit den zur Zielposition abweichungsstärkeabhängigen grafischen Zeichen oder verbalen Signalen, ansteuernd vorgesehen ist.

11. Blutdruckmeßgerät nach Anspruch 10, **dadurch gekennzeichnet, daß** die Kontrolleinheit oder die Anzeigeeinrichtung (5) derart ausgebildet sind, daß auf der Anzeigeeinrichtung (5) ein Oberkörper mit einem angewinkelten Unterarm darstellbar ist.

12. Blutdruckmeßgerat nach einem der Ansprüche 10 oder 11, **dadurch gekennzeichnet, daß** die Kontrolleinheit und/oder die Anzeigeeinrichtung (5) derart ausgebildet sind, daß die Benutzerführung zur Erreichung der Zielposition auf der Anzeigeeinrichtung (5) einen passiven und einen animierten Bestandteil aufweist.

13. Blutdruckmeßgerät nach zumindest einem der Ansprüche 10 bis 12, **dadurch gekennzeichnet, daß** die Anzeigeeinrichtung (5) neben der erforderlichen Benutzeraktion die einzunehmende Meßposition und die aktuelle Meßposition darstellt.

14. Blutdruckmeßgerät nach zumindest einem der Ansprüche 12 oder 13, **dadurch gekennzeichnet, daß** die Anzeigeeinrichtung (5) derart ausgebildet ist, daß als passiver Bestandteil die einzuhehmende Meßposition mit einem Oberkörper und einem angewinkelten Unterarm anzeigbar ist und daß die Kontrolleinheit derart ausgebildet ist, daß innerhalb des passiven Bestandteils ein Unterarm abhängig vom Ergebnis der Auswertung der aktuell erfaßten Position der Manschette (4) anzeigbar ist, der die aktuelle Meßposition wiedergibt.

15. Blutdruckmeßgerat nach zumindest einem der Ansprüche 10 bis 14, **dadurch gekennzeichnet, daß** ein Mikroprozessor zur Steuerung und Regelung des Meßablaufs derart ausgebildet ist, daß die Blutdruckmessung dann automatisch startet, wenn die Zielposition für die korrekte Meßhaltung erreicht oder eine bestimmte Zeit verstrichen ist.

16. Blutdruckmeßgerat nach Anspruch 10, **dadurch gekennzeichnet, daß** die Kontrolleinheit derart ausgebildet ist, daß die Anzeigeeinrichtung (5) abhängig vom Ergebnis der Auswertung der aktuell erfaßten Position der Manschette (4) eine Textanweisung oder Sprachanweisung und damit eine unmittelbare Anweisung für die zu positionierenden Körperteile ausgibt.

17. Blutdruckmeßgerät nach einem der Ansprüche 10 bis 16, **dadurch gekennzeichnet, daß** die Kontrolleinheit und/oder die Anzeigeeinrichtung (5) derart ausgebildet sind, daß die grafische Darstellung der einzunehmenden Meßposition den auf der Anzeigeeinrichtung (5) linksseitigen Unterarm angewinkelt zeigt.

## Claims

1. A method for blood pressure measurement in which a cuff (4) is placed on the wrist or a finger of a user, a position of the cuff (4) is detected with the help of a position detecting device, the detected position of the cuff (4) is analyzed, the display of graphic characters or the output of verbal signals is controlled by a control unit for a user guidance and the results of the blood pressure measurement as well as the graphic characters or verbal signals for the user guidance are output on a display device (5), whereby the graphic characters or verbal signals about imminent required user actions are output as a function of the result of the analysis of the position of the cuff (4) as currently detected so that a target position is reached, **characterized in that** the control unit controls the display device (5) with graphic characters or verbal signals which depend on the extent of deviation from the target position via the direction of movement with respect to the target position to be assumed whereby different graphic characters or verbal signals are output with different deviations from the target position.

2. The method according to Claim 1, **characterized in that** a torso with a bent forearm is displayed on the display device (5).

3. The method according to any one of Claims 1 or 2, **characterized in that** the user guidance to reach the target position on the display device (5) has a passive component and an animated component.

4. The method according to at least one of Claims 1 through 3, **characterized in that** the display device (5) illustrates the measurement position to be assumed and the current measurement position in addition to the user action required, which is shown with animation in particular.

5. The method according to at least one of Claims 3 or 4, **characterized in that** the measurement position to be assumed is displayed with a torso and a bent forearm as a passive component and within the passive component an animated forearm is displayed depending on the result of the analysis of the position currently detected for the cuff (4), reflecting the current measurement position.

6. The method according to at least one of Claims 1 to 5, **characterized in that** the blood pressure measurement is started automatically when the target position for the correct measurement position is reached or a certain period of time has elapsed.

7. The method according to at least one of Claims 1 to 6, **characterized in that** the start of the blood pressure measurement and/or the target position for the correct measurement posture is displayed by an acoustic signal.

8. The method according to Claim 7, **characterized in that** depending on the circumstances of how the start of the blood pressure measurement comes about, a different acoustic signal is delivered.

9. The method according to Claim 1, **characterized in that** the display device (5) outputs a text instruction or voice instructions depending on the result of the analysis of the position of the cuff (4) currently detected and in doing so outputs a direct instruction for the body parts to be positioned.

10. A blood pressure meter having a cuff (4) for a blood pressure measurement on the wrist or finger of a user, a posture detection device for detecting the position of the cuff (4), an analyzer unit for analyzing the detected position of the cuff (4), a control unit for controlling the display of graphic characters or verbal signals for user guidance and a display device (5) which outputs the results of the blood pressure measurement and the graphic characters or verbal signs for the user guidance, whereby the display device (5) is provided for outputting graphic characters or verbal signs for the user guidance about an imminent required user action depending on the results of the analysis of the currently detected position of the cuff (4) so that a target position can be reached, **characterized in that** the display device (5) can be triggered with different graphic characters or verbal signals that depend on the extent of the deviation from the target position and the display device is provided for outputting them and the control unit is provided for triggering the display device (5) with which graphic characters or verbal signals that depend on the extent of the deviation from the target position.

11. The blood pressure meter according to Claim 10, **characterized in that** the control unit or the display device (5) are designed so that a torso with a bent forearm can be displayed on the display device (5).

12. The blood pressure meter according to any one of Claims 10 or 11, **characterized in that** the control unit and/or the display device (5) are designed so that the user guidance for achieving the target position on the display device (5) has a passive component and an animated component.

13. The blood pressure meter according to at least one of Claims 10 through 12, **characterized in that** the display device (5) represents the measurement position to be assumed and the current measurement position in addition to the required user action.

14. The blood pressure meter according to at least one of Claims 12 or 13, **characterized in that** the display device (5) is designed so that the measurement position to be assumed with a torso and a bent forearm can be displayed as a passive component, and the control unit is designed so that a forearm can be displayed within the passive component as a function of the result of the analysis of the position of the cuff (4) as currently detected, reproducing the current measurement position.

15. The blood pressure meter according to at least one of Claims 10 through 14, **characterized in that** a microprocessor for controlling and regulating the measurement process is designed so that the blood pressure measurement starts automatically when the target position for the correct measurement position is reached or a certain period of time has elapsed.

16. The blood pressure meter according to Claim 10, **characterized in that** the control unit is designed so that the display device (5) outputs a text instruction or a voice instruction and thus outputs a direct instruction for the body parts to be positioned and does so as a function of the result of the analysis of the currently detected position of the cuff (4).

17. The blood pressure meter according to any one of Claims 10 through 16, **characterized in that** the control unit and/or the display device (5) are designed so that the graphic display of the measurement position to be assumed displays the forearm on the left side of the display device (5) in a bent position.

## Revendications

1. Procédé de mesure de la tension artérielle, dans lequel un manchon (4) est posé au poignet ou au doigt d'un utilisateur, une position du manchon (4) est enregistrée au moyen d'un dispositif d'enregistrement de position, la position enregistrée du manchon(4) est exploitée, la représentation de caractères graphiques ou l'émission de signaux verbaux pour un guidage de l'utilisateur est commandée par une unité de commande et aussi bien les résultats de la mesure de tension artérielle que les caractères graphiques ou les signaux verbaux pour le guidage de l'utilisateur sont émis sur un dispositif d'affichage (5), les caractères graphiques ou les signaux verbaux étant émis grâce à des actions nécessaires imminentes de l'utilisateur en fonction du résultat de l'exploitation de la position momentanément enregistrée du manchon (4) afin d'atteindre une position ciblée, **caractérisé en ce que** l'unité de commande amorce le dispositif d'affichage (5) par des caractères graphiques ou des signaux verbaux dépendant de l'importance de l'écart par rapport à la position ciblée relatifs au sens de mouvement par rapport à la position ciblée à atteindre, des caractères graphiques ou des signaux verbaux différents étant émis en cas d'écarts d'une importance différente par rapport à la position ciblée.

2. Procédé selon la revendication 1, **caractérisé en ce que** le dispositif d'affichage (5) permet de représenter un haut du corps avec un avant-bras replié.

3. Procédé selon l'une quelconque des revendications 1 ou 2, **caractérisé en ce que** le guidage de l'utilisateur pour atteindre la position ciblée sur le dispositif d'affichage (5) comporte une composante passive et une composante animée.

4. Procédé selon au moins l'une quelconque des revendications 1 à 3, **caractérisé en ce que** le dispositif d'affichage (5), outre l'action nécessaire de l'utilisateur qui est notamment représentée en animation, représente la position de mesure à prendre et la position de mesure momentanée.

5. Procédé selon au moins l'une quelconque des revendications 3 ou 4, **caractérisé en ce que** s'affiche comme composante passive la position de mesure à prendre avec un haut du corps et un avant-bras replié et que s'affiche dans la composante passive un avant-bras animé en fonction du résultat de l'exploitation de la position momentanément enregistrée du manchon (4), cet avant-bras représentant la position de mesure momentanée.

6. Procédé selon au moins l'une quelconque des revendications 1 à 5, **caractérisé en ce que** la mesure de la tension artérielle ne démarre automatiquement qu'une fois que la position ciblée pour la position de mesure correcte est atteinte ou qu'une certaine durée s'est écoulée.

7. Procédé selon au moins l'une quelconque des revendications 1 à 6, **caractérisé en ce que** le démarrage de la mesure de la tension artérielle et/ou la position ciblée pour une position de mesure correcte sont indiqués par un signal acoustique.

8. Procédé selon la revendication 7, **caractérisé en ce que**, suivant les circonstances de l'obtention du démarrage de la mesure de la tension artérielle, un signal acoustique chaque fois différent est émis.

9. Procédé selon la revendication 1, **caractérisé en ce que** le dispositif d'affichage (5), en fonction du résultat de l'exploitation de la position momentanément enregistrée du manchon (4), émet une instruction en texte ou une instruction vocale et ainsi une instruction directe pour les parties du corps à positionner.

10. Appareil de mesure de la tension artérielle comportant un manchon (4) pour mesurer la tension artérielle au poignet ou au doigt d'un utilisateur, un dispositif d'enregistrement de position pour enregistrer la position du manchon (4), une unité d'exploitation pour exploiter la position enregistrée du manchon (4), une unité de commande pour commander la représentation de caractères graphiques ou de signaux verbaux pour un guidage de l'utilisateur et une unité d'affichage (5) émettant les résultats de la mesure de la tension artérielle et les caractères graphiques ou les signaux verbaux pour le guidage de l'utilisateur, le dispositif d'affichage (5) étant prévu pour émettre des caractères graphiques ou des signaux verbaux pour le guidage de l'utilisateur grâce à des actions nécessaires imminentes de l'utilisateur en fonction du résultat de l'exploitation de la position momentanément enregistrée du manchon (4) afin de pouvoir atteindre une position ciblée, **caractérisé en ce que** le dispositif d'affichage (5) est prévu pour être amorcé par différents caractères graphiques ou signaux verbaux en fonction de l'importance de l'écart par rapport à la position ciblée et pour les émettre et que l'unité de commande est prévue pour amorcer l'unité d'affichage (5) par les caractères graphiques ou signaux verbaux en fonction de l'importance de l'écart par rapport à la position ciblée.

11. Appareil de mesure de la tension artérielle selon la revendication 10, **caractérisé en ce que** l'unité de commande ou le dispositif d'affichage (5) est réalisé de manière à ce que puisse être représenté sur le dispositif d'affichage (5) un haut du corps avec un avant-bras replié.

12. Appareil de mesure de la tension artérielle selon l'une quelconque des revendications 10 ou 11, **caractérisé en ce que** l'unité de commande et/ou le dispositif d'affichage (5) sont réalisés de manière à ce que le guidage de l'utilisateur pour atteindre la position ciblée sur le dispositif d'affichage (5) comprenne une composante passive et une composante animée.

13. Appareil de mesure de la tension artérielle selon au moins l'une quelconque des revendications 10 à 12, **caractérisé en ce que** le dispositif d'affichage (5), outre l'action nécessaire de l'utilisateur, représente la position de mesure à prendre et la position de mesure momentanée.

14. Appareil de mesure de la tension artérielle selon au moins l'une quelconque des revendications 12 ou 13, **caractérisé en ce que** le dispositif d'affichage (5) est réalisé de manière à ce que puisse s'afficher comme composante passive la position de mesure à prendre avec un haut du corps et un avant-bras replié et que l'unité de commande est réalisée de manière à ce que, dans la composante passive, un avant-bras puisse s'afficher en fonction du résultat de l'exploitation de la position momentanément enregistrée du manchon (4), cet avant-bras représentant la position de mesure momentanée.

15. Appareil de mesure de la tension artérielle selon au moins l'une quelconque des revendications 10 à 14, **caractérisé en ce qu'**un microprocesseur permettant de commander et de réguler le déroulement de la mesure est réalisé de manière à ce que la mesure de la tension artérielle démarre automatiquement une fois que la position ciblée pour la position de mesure correcte est atteinte ou qu'une certaine durée s'est écoulée.

16. Appareil de mesure de la tension artérielle selon la revendication 10, **caractérisé en ce que** l'unité de commande est réalisée de manière à ce que le dispositif d'affichage (5), en fonction du résultat de l'exploitation de la position momentanément enregistrée du manchon (4), émet une instruction en texte ou une instruction vocale et ainsi une instruction directe pour les parties du corps à positionner.

17. Appareil de mesure de la tension artérielle selon l'une quelconque des revendications 10 à 16, **caractérisé en ce que** l'unité de commande et/ou le dispositif d'affichage (5) sont réalisés de manière à ce que la représentation graphique de la position de mesure à prendre montre l'avant-bras replié du côté gauche sur le dispositif d'affichage (5).
